(19) Europäisches Patentamt
European Patent Office
Office européen des brevets

(11) Numéro de publication: **0 097 578**
**B1**

(12) **FASCICULE DE BREVET EUROPEEN**

(45) Date de publication du fascicule du brevet: **11.09.85**

(51) Int. Cl.[4]: **C 07 C 51/09**, C 07 C 59/245, C 07 C 67/333, C 07 C 69/675

(21) Numéro de dépôt: **83401216.3**

(22) Date de dépôt: **14.06.83**

# (54) Procédé de préparation de l'acide 3-méthyl 3-hydroxy glutarique et produit intermédiaire nécessaire à cette préparation.

(30) Priorité: **17.06.82 FR 8210597**

(43) Date de publication de la demande: **04.01.84 Bulletin 84/1**

(45) Mention de la délivrance du brevet: **11.09.85 Bulletin 85/37**

(84) Etats contractants désignés: **AT BE CH DE GB IT LI NL SE**

(56) Documents cités: **US - A - 3 818 080**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Gigliotti Giuseppe, 5, rue A. Baudin no 464, F-75011 Paris (FR)**
Inventeur: **Roul, Jean-Michel, Résidence Périchelles 4, square des Aulnes, F-77500 Chelles (FR)**

(74) Mandataire: **Tonnellier, Marie-José, ROUSSEL-UCLAF 111, route de Noisy Boîte Postale no.9, F-93230 Romainville (FR)**

**EP 0 097 578 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La presente invention concerne un procédé de préparation de l'acide 3-méthyl 3-hydroxy glutarique de formule I:

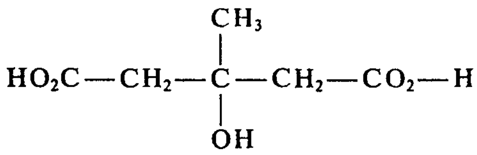

(I)

L'utilisation de ce produit en thérapeutique est connue. C'est ainsi que le brevet américain 3 629 449 décrit l'application de l'acide 3-méthyl 3-hydroxy glutarique comme hypocholestérolémiant et hypolipémiant. Le brevet américain 4 105 794 décrit l'utilisation de cet acide dans le traitement des calculs biliaires.

Des procédés de préparation de ce produit sont également connus dans la littérature.

Dans le brevet français n° 2 411 822 est décrit un procédé d'oxydation par un catalyseur d'interface du produit de formule (A):

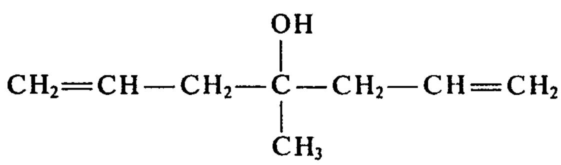

(A)

Ce procédé ne semble pas donner de rendements satisfaisants.

Une coupure oxydante du produit A est décrite notamment dans la référence Bioch. Préparations 6, 25 (1958).

Cependant l'utilisation de l'ozone, si elle présente toujours des difficultés d'adaptation à l'échelle industrielle, semble en l'espèce particulièrement dangereuse. Dans la revue Chem. Eng. News 51 (6) 29 le Dr. Milles rapporte en effet une explosion survenue lors de l'utilisation de cette méthode.

On peut enfin noter une méthode récente décrite dans Synthesis, 10, d'octobre 1981 p. 791. Cette méthode est la suivante:

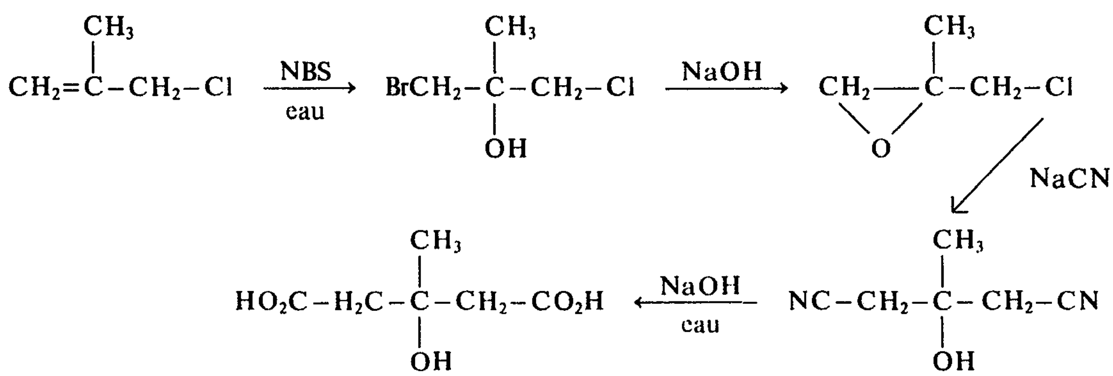


Cette méthode apparaît cependant assez coûteuse du fait des réactifs employés et des rendements moyens observés.

Le procédé, objet de la présente demande, est caractérisé en ce que l'on fait réagir un produit de formule II:

$$CH_3-CO-R \qquad (II)$$

dans laquelle R représente un radical alkoxy ayant de 1 à 6 atomes de carbone, un radical formyloxy, un radical acyloxy ayant de 2 à 18 atomes de carbone ou un atome d'halogène, avec au moins deux équivalents d'un produit de formule III:

$$X-CH_2-CO_2-R_1 \qquad (III)$$

dans laquelle X représente un atome de métal alcalin et $R_1$ représente un radical alkyle ayant de 1 à 6 atomes de carbone, pour obtenir un produit de formule IV:

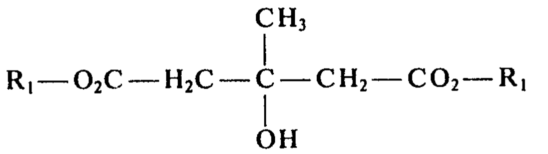

(IV)

produit de formule IV que l'on transforme en acide de formule I par saponification ou décomposition thermique éventuellement catalysée.

Dans la formule II, le radical alkoxy que peut représenter R peut être, notamment, formé à partir des radicaux alkyles suivants: un radical méthyle, éthyle, propyle, isopropyle, butyle, isobutyle, butyle secondaire, tert-butyle, pentyle, isopentyle, pentyle secondaire, term-amyle, néopentyle, hexyle.

Le radical acyloxy que peut également représenter R peut être un radical acétyloxy, propionyloxy, butyryloxy, isobutyryloxy, pivaloyloxy, undécanoyloxy, acryloyloxy, crotonoyloxy, cyclopropyl carbonyloxy, cyclobutyl carbonyloxy ou cyclopentylcarbonyloxy, cyclohexylacétyloxy, cyclohexylpropionyloxy, benzoyloxy, phénylacétyloxy, phénylpropionyloxy.

L'atome d'halogène peut être un atome de chlore, de brome ou d'iode.

Parmi les métaux alcalins que peut représenter le radical X dans le produit de formule III, on préfère les atomes de lithium, sodium ou potassium. Les valeurs de $R_1$ peuvent être choisies dans la liste de radicaux alkyles énoncés ci-dessus.

La saponification des produits de formule IV est effectuée de préférence par une base telle que la soude, la potasse ou la baryte. La saponification est alors bien entendu suivie d'une réacidification utilisant un acide tel que l'acide chlorhydrique, ou sulfurique.

La décomposition thermique peut être effectuée par chauffage à une température de l'ordre de 180 à 250° C, de préférence autour de 180° C. La décomposition thermique peut de préférence être catalysée par un acide tel que l'acide chlorhydrique, on chauffe alors de préférence au reflux d'une solution aqueuse.

La présente invention a particulièrement pour objet un procédé de préparation de l'acide 3-hydroxy 3-méthyl glutarique caractérisé en ce que l'on fait réagir un produit de formule IIa:

$$CH_3-CO-Ra \qquad \text{(IIa)}$$

dans laquelle Ra représente un radical alkoxy ayant de 1 à 4 atomes de carbone, un radical alkanoyloxy ayant de 2 à 4 atomes de carbone ou un atome d'halogène, avec au moins deux équivalents d'un produit de formule IIIa:

$$X'-CH_2-CO_2-R_{1a} \qquad \text{(IIIa)}$$

dans laquelle X' représente un atome de lithium et $R_{1a}$ représente un radical alkyle ayant de 1 à 4 atomes de carbone pour obtenir un produit de formule IVa:

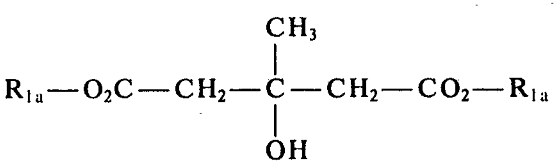

(IVa)

produit de formule IVa que l'on transforme en acide de formule I par saponification ou décomposition thermique avec catalyse acide.

Plus particulièrement encore, le procédé de préparation de l'acide 3-méthyl 3-hydroxy glutarique, objet de la présente invention, est caractérisé en ce que l'on met en oeuvre le procédé en utilisant un produit de formule IIa dans laquelle Ra représente un radical méthoxy, éthoxy, ou acétyloxy ou un atome de chlore et un produit de formule IIIa dans laquelle X' représente un atome de lithium et $R_{1a}$ représente un radical méthyle, éthyle ou tert. butyle et en ce que la saponification du produit de formule IVa obtenu est réalisée en présence de soude aqueuse.

On préfère tout particulièrement le procédé utilisant un produit de formule IIa dans laquelle $R_a$ représente un radical acétyloxy ou un atome de chlore et un produit de formule IIIa dans laquelle X' représente un atome de lithium et $R_{1a}$ représente un radical tert-butyle.

Les conditions opératoires nécessaires à la mise en oeuvre du procédé sont illustrées de manière indicatives et non limitatives dans les exemples ci-dessous.

Le procédé est cependant mis en oeuvre préférentiellement dans les conditions suivantes: la réaction du produit de formule IIa avec le produit de formule IIIa est effectuée à une température comprise entre 0° et −80° C dans un solvant ou un mélange de solvants choisis dans le groupe formé par le tétrahydrofuranne, le diméthyl formamide, l'hexaméthylphosphotriamide et le méthylcyclohexane.

On peut également préciser que dans un mode préféré d'exécution, le produit de formule III:

$X-CH_2-CO_2-R_1$ (III)

dans laquelle X et $R_1$ ont la signification indiquée ci-dessus, ou le produit de formule IIIa:

$X'-CH_2-CO_2-R_{1a}$ (IIIa)

dans laquelle X' et $R_{1a}$ ont la signification indiquée ci-dessus, sont préparés par action, dans un solvant, d'une base forte sur l'acétate d'alkyle correspondant et en ce que le produit de formule III ou IIIa obtenu en solution est mis directement en présence du produit de formule II ou IIa.

La base forte que l'on utilise est très préférentiellement un dialkyl amidure de lithium préparé in situ par action du butyllithium sur une dialkylamine. On préfère le diisopropyl amidure de lithium préparé in situ par actino du butyllithium en solution cyclohexanique sur la diisopropylamine.

On peut cependant utiliser d'autres bases fortes telles que le tert-butylate de potassium ou l'amidure de sodium.

Un mode d'exécution préféré du présent procédé consiste alors à ajouter le produit de formule II ou IIa dans la solution constituée par le produit de formule III ou IIIa dans un solvant ou un mélange de solvants choisis dans le groupe formé par le tétrahydrofuranne, le diméthyl formamide, l'hexaméthylphosphotriamide, le méthylcyclohexane.

Les conditions préférentielles de température, sont alors celles indiquées ci-dessus.

On opère très préférentiellement à une température de l'ordre de −40° C.

L'introduction du produit de formule II ou IIa dans la solution du produit de formule III ou IIIa est de préférence effectuée lentement. Une durée allant jusqu'à 2 heures est possible. Il semble néanmoins que l'introduction peut être réduite à 30 minutes environ. La réaction elle-même entre les produits peut, selon les conditions employées, nécessiter un contact de l'ordre de 10 à 15 heures. Dans les conditions préférentielles de températures indiquées ci-dessus, une durée de l'ordre de 2 heures apparaît généralement suffisante.

La présente invention a enfin pour objet, à titre de produit industriel nouveau et notamment à titre de produit industriel nouveau et notamment à titre de produit industriel nouveau nécessaire pour la mise en oeuvre du procédé indiqué ci-dessus, l'ester di-terbutylique de l'acide 3-méthyl 3-hydroxy glutarique.

Les exemples suivants illustrent l'invention sans toutefois la limiter.

## Exemple 1

### Ester di-terbutylique de l'acide 3-méthyl 3-hydroxy glutarique

On refroidit à −40° C sous argon 500 cm3 de tétrahydrofuranne puis introduit en 5 minutes 187 g d'une solution cyclohexanique de butyllithium à 19,5% (soit 36,4 g de butyllithium) puis ajoute à cette température, en 5 minutes, 57,5 g de diisopropylamine. On agite 15 minutes à −40° C puis rajoute à cette température, en 30 minutes, 66 g d'acétate de tert-butyle. On agite encore 30 minutes à −40° C puis introduit en 30 minutes, à cette température, 27,5 g d'acétate d'éthyle (≃0,55 mole par mole d'acétate de tertbutyle). On agite deux heures à −40° C puis ramène la température à 20° C sous pression réduite, on distille à cette température et amène à sec.

On reprend le résidu par 200 cm3 de chlorure de méthylène, refroidit à 0° C et ajoute à cette température 100 cm3 d'eau. On décante, extrait la phase aqueuse par du chlorure de méthylène. On réunit les phases organiques, les amène à pH = 1 avec de l'acide chlorhydrique 22° Bé. On lave à l'eau, sèche, filtre et concentre à sec sous pression réduite.

On obtient un liquide légèrement coloré que l'on rectifie sous 1 mm de mercure. On recueille la fraction constituée par le produit attendu ($Eb_1$: 95° C ± 2° C) soit 25 g.

| | | |
|---|---|---|
| Analyse: | $C_{14}H_{26}O_5$ | |
| Calculé: | C% 61,29 | H% 9,55 |
| Trouvé: | C% 61,3 | H% 9,5 |

## Exemple 2

### Ester di-terbutylique de l'acide 3-méthyl 3-hydroxy glutarique

On opère dans des conditions identiques à celles indiquées à l'exemple 1 en remplaçant l'acétate d'éthyle par 0,55 mole d'acétate de méthyle. On obtient un résultat sensiblement identique.

Exemple 3

Ester di-terbutylique de l'acide 3-méthyl 3-hydroxy glutarique

On opère dans des conditions identiques à celles indiquées à l'exemple 1, en effectuant les modifications opératoires suivantes:

A) L'introduction de l'acétate d'éthyle puis l'agitation sont effectuées à −70° C.
B) L'introduction de l'acétate d'éthyle puis l'agitation sont effectuées à −20° C.
C) L'introduction de l'acétate d'éthyle puis l'agitation sont effectuées à 0° C.
D) On remplace le tétrahydrofuranne utilisé depuis le début de la réaction:
   - par un mélange tétrahydrofuranne-hexaméthyl phosphotriamide;
   - par un mélange tétrahydrofuranne-diméthylformamide;
   - par un mélange tétrahydrofuranne-méthylcyclohexane.
E) L'acétate de tert-butyle est introduit en 1 heure 30 minutes puis l'agitation est poursuivie 1 heure; l'acétate d'éthyle est introduit en 2 heures puis l'agitation est poursuivie 15 heures.

Exemple 4

Ester di-terbutylique de l'acide 3-méthyl 3-hydroxy glutarique

On opère dans les conditions identiques à celles indiquées à l'exemple 1, en remplaçant l'acétate d'éthyle par:

A) l'acétate de tert-butyle
B) l'anhydride acétique.

Exemple 5

Ester di-éthylique de l'acide 3-méthyl 3-hydroxy glutarique

On opère dans les conditions descrites à l'exemple 1 en remplaçant l'acétate de tert-butyle par l'acétate d'éthyle. L'acétate d'éthyle utilisé ensuite à l'exemple 1 est remplacé par

A) l'anhydride acétique
B) le chlorure d'acétyle.

Exemple 6

Ester di-méthylique de l'acide 3-méthyl 3-hydroxy glutarique

On opère dans les conditions descrites à l'exemple 1 en remplaçant l'acétate de tert-butyle par l'acétate d'méthyle et l'acétate d'éthyle par le chlorure d'acétyle.

Exemple 7

Ester di-terbutylique de l'acide 3-méthyl 3-hydroxy glutarique

On introduit à − 40° C et en 15 minutes, 935 g d'une solution hexanique à 19,5% de butyllithium (soit 182,3 g de butyllithium) dans 2500 cm3 de tétrahydrofuranne refroidi à −40° C sous azote.

On introduit, à cette même température de −40° C et en 20 minutes, 287,5 g de diisopropylamine. On laisse en contact 15 minutes à −40° C puis introduit en 30 minutes à −40° C, 330 g d'acétate de tert-butyle. On laisse 30 minutes en contact à −40° C puis introduit en 30 minutes à −40° C, 122,6 g de chlorure d'acétyle.

On agite deux heures à −40° C puis ramène la température à +20° C.

On distille à sec sous vide sans dépasser 25° C. On rajoute à 20° C, 1000 cm3 de chlorure de méthylène, sous agitation, on rajoute sans dépasser 20° C, 500 cm3 d'eau glacée puis ajoute 200 cm3 d'acide chlorhydrique 22° Bé pour amener à pH = 1. On décante, extrait la phase aqueuse par deux fois 150 cm3 de chlorure de méthylène. On réunit les phases organiques, les lave par 3 fois 500 cm3 d'eau déminéralisée. On sèche, filtre, rince au chlorure de méthylène et concentre à sec sous vide.

On obtient 337,7 g de produit.

### Exemple 8

### Acide 3-méthyl 3-hydroxy glutarique

On chauffe 30 minutes à 180° C, 6,4 g d'ester di-terbutylique de l'acide 3-méthyl 3-hydroxy glutarique. On refroidit à 20° C, le di-acide formé cristallise.

On obtient 3,45 g de produit que l'on recristallise dans l'acétate d'éthyle.

On obtient un produit pur, F = 108° C

### Exemple 9

### Acide 3-méthyl 3-hydroxy glutarique

On chauffe 5 heures au reflux sous vive agitation une émulsion constituée par 333,7 g d'ester di-terbutylique de l'acide 3-méthyl 3-hydroxy glutarique, 670 cm3 d'eau déminéralisée et 6,7 cm3 d'acide chlorhydrique 22° Bé.

On distille ensuite à pression ordinaire, 166 cm3 d'eau. On distille ensuite le volume résiduel sous vide et à 40° C environ. On sèche le produit obtenu à 40° C et obtient 144,8 g de produit attendu.

Ce produit peut être purifié comme suit: on porte au reflux un mélange constitué par les 144,8 g obtenus ci-dessus dans 600 cm3 d'acétate d'éthyle et ajoute 14 g de charbon actif. Après 10 minutes de reflux on filtre à chaud et rince par 200 cm3 d'acétate d'éthyle bouillant.

Sous agitation on refroidit à —10° C et conserve 30 minutes à cette température. On essore et lave par 100 cm3 d'acétate d'éthyle à —10° C.

On sèche en étuve à 40° C et obtient 112 g de produit purifié, F = 108° C.

On obtient un deuxième jet en concentrant les liqueurs-mères à 100 cm3 environ. On agite 30 minutes à —10° C, essore et lave par 20 cm3 d'acétate d'éthyle à —10° C. On sèche à 40° C et obtient 9,72 g de produit.

On dissout ce produit dans 48 cm3 d'acétate d'éthyle au reflux, refroidit à —10° C, agite 30 minutes à —10° C, essore et lave par 10 cm3 d'acétate d'éthyle à —10° C. On sèche à 40° C et isole un deuxième jet de 7,68 g de produit pur. F = 108° C.

### Exemple 10

### Acide 3-méthyl 3-hydroxy glutarique

On rajoute en une seule fois à 20° C, 146 g de soude caustique en écailles à un mélange de 333,7 g d'ester diterbutylique de l'acide 3-méthyl 3-hydroxy glutarique dans 2000 cm3 d'eau puis porte 5 heures au reflux sous vive agitation l'émulsion obtenue.

On distille ensuite 200 cm3 de solvant sous pression ordinaire. On refroidit vers 50° C et distille 1400 cm3 d'eau sous pression réduite. On refroidit ensuite vers 20° C et amène à pH = 0,5 par addition de 292 cm3 d'acide chlorhydrique 22° Bé. On concentre ensuite à sec sous pression réduite et obtient, après séchage, 327,2 g de produit contenant du chlorure de sodium.

On porte 15 minutes à reflux un mélange de 327,2 g de produit précédant et 800 cm3 d'acétate d'éthyle dans lequel on a rajouté 12 g de charbon actif. On filtre à chaud et empâte par 2 fois avec 200 cm3 d'acétate d'éthyle bouillant. On concentre le filtrat sous pression réduite jusqu'à un volume de 600 cm3 en chauffant avec un bain extérieur à 40° C.

On refroidit à —10° C, agite 30 minutes à cette température essore et lave par 200 cm3 d'acétate d'éthyle à —10° C. On sèche en étuve à 40° C environ et obtient 104 g de produit, F = 109° C.

On obtient un second jet du produit en opérant comme suit:

On concentre les liqueurs-mères d'acétate d'éthyle à environ 60 cm3. On refroidit à —10° C et agite 30 minutes à —10° C. On essore et lave par 10 cm3 d'acétate d'éthyle à —10° C. On sèche en étuve à 40° C et obtient 12,8 g de produit qui est ensuite redissout dans 64 cm3 d'acétate d'éthyle. On refroidit à —10° C, agite 30 minutes à —10° C, essore et lave par 12,8 cm3 d'acétate d'éthyle à —10° C. On sèche en étuve à 40° C et obtient 10 g de produit pur supplémentaire.

**Revendications pour les Etats contractants: BE, CH, DE, GB, IT, LI, NL, SE**

1. Procédé de préparation de l'acide 3-méthyl 3-hydroxy glutarique de formule I:

$$HO_2C-CH_2\overset{\displaystyle CH_3}{\underset{\displaystyle OH}{\overset{|}{\underset{|}{C}}}}-CH_2-CO_2H \qquad (I)$$

caractérisé en ce que l'on fait réagir un produit de formule II:

$$CH_3-CO-R \qquad (II)$$

dans laquelle R représente un radical alkoxy ayant de 1 à 6 atomes de carbone, un radical formyloxy, un radical acyloxy ayant de 2 à 18 atomes de carbone ou un atome d'halogène, avec au moins deux équivalents d'un produit de formule III:

$$X-CH_2-CO_2-R_1 \qquad (III)$$

dans laquelle X représente un atome de métal alcalin et $R_1$ représente un radical alkyle ayant de 1 à 6 atomes de carbone, pour obtenir un produit de formule IV:

$$R_1-O_2C-H_2C-\overset{\displaystyle CH_3}{\underset{\displaystyle OH}{\overset{|}{\underset{|}{C}}}}-CH_2-CO_2-R_1 \qquad (IV)$$

produit de formule IV que l'on transforme en acide de formule I par saponification ou décomposition thermique éventuellement catalysée.

2. Procédé selon la revendication 1 de préparation de l'acide 3-méthyl 3-hydroxy glutarique, caractérisé en ce que l'on fait réagir un produit de formule IIa:

$$CH_3-CO-R_a \qquad (IIa)$$

dans laquelle $R_a$ représente un radical alkoxy ayant de 1 à 4 atomes de carbone, un radical alkanoyloxy ayant de 2 à 4 atomes de carbone ou un atome d'halogène, avec au moins deux équivalents d'un produit de formule IIIa:

$$X'-CH_2-CO_2-R_{1a} \qquad (IIIa)$$

dans laquelle X' représente un atome de lithium et $R_{1a}$ représente un radical alkyle ayant de 1 à 4 atomes de carbone pour obtenir un produit de formule IVa:

$$R_{1a}-O_2C-CH_2-\overset{\displaystyle CH_3}{\underset{\displaystyle OH}{\overset{|}{\underset{|}{C}}}}-CH_2-CO_2-R_{1a} \qquad (IVa)$$

produit de formule IVa que l'on transforme en acide de formule I par saponification ou décomposition thermique avec catalyse acide.

3. Procédé selon la revendication 2 de préparation de l'acide 3-méthyl 3-hydroxy glutarique de formule I, caractérisé en ce que l'on met en oeuvre le procédé en utilisant un produit de formule IIa dans laquelle $R_a$ représente un radical méthoxy, éthoxy, ou acétyloxy ou un atome de chlore et un produit de formule IIIa dans laquelle X' représente un atome de lithium et $R_{1a}$ représente un radical méthyle, éthyle ou tert-butyle et en ce que la saponification du produit de formule IVa obtenu est réalisée en présence de soude aqueuse.

4. Procédé selon l'une quelconque des revendications 2 ou 3, caractérisé en ce que l'on met en oeuvre le procédé en utilisant un produit de formule IIa dans laquelle $R_a$ représente un radical acétyloxy ou un atome de chlore et un produit de formule IIIa dans laquelle X' représente un atome de lithium et $R_{1a}$ représente un radical tert-butyle.

5. Procédé selon l'une quelconque des revendications 2 à 4, caractérisé en ce que la réaction du produit de formule IIa avec le produit de formule IIIa est effectuée à une température comprise entre 0° et −80° C dans un solvant ou un mélange de solvants choisis dans le groupe formé par le tétrahydrofuranne, le diméthyl formamide, l'hexaméthylphosphotriamide et le méthylcyclohexane.

6. Procédé selon l'une quelconque des revendications 2 à 5, caractérisé en ce que le produit de formule III:

$$X-CH_2-CO_2-R_1 \qquad \text{(III)}$$

dans laquelle X et $R_1$ ont la signification indiquée à la revendication 1, ou le produit de formule IIIa:

$$X'-CH_2-CO_2-R_{1a} \qquad \text{(IIIa)}$$

dans laquelle X' et $R_{1a}$ ont la signification indiquée à la revendication 2, sont préparés par action, dans un solvant, d'une base forte sur l'acétate d'alkyle correspondant et en ce que le produit de formule III ou IIIa obtenu en solution est mis directement en présence du produit de formule II ou IIa.

7. Procédé selon l'une quelconque des revendications 1 à 6, caractérisé en ce que l'on ajoute le produit de formule II ou IIa dans la solution constituée par le produit de formule III ou IIIa dans un solvant ou un mélange de solvants choisis dans le groupe formé par le tétrahydrofuranne, le diméthyl formamide, l'hexaméthylphosphotriamide, le méthylcyclohexane.

8. A titre de produit industriel nouveau, l'ester diterbutylique de l'acide 3-méthyl 3-hydroxy glutarique.

**Revendications pour l'Etat contractant: AT**

1. Procédé de préparation de l'acide 3-méthyl 3-hydroxy glutarique de formule I:

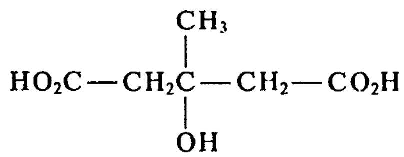
(I)

caractérisé en ce que l'on fait réagir un produit de formule II:

$$CH_3-CO-R \qquad \text{(II)}$$

dans laquelle R représente un radical alkoxy ayant de 1 à 6 atomes de carbone, un radical formyloxy, un radical acyloxy ayant de 2 à 18 atomes de carbone ou un atome d'halogène, avec au moins deux équivalents d'un produit de formule III:

$$X-CH_2-CO_2-R_1 \qquad \text{(III)}$$

dans laquelle X représente un atome de métal alcalin et $R_1$ représente un radical alkyle ayant de 1 à 6 atomes de carbone, pour obtenir un produit de formule IV:

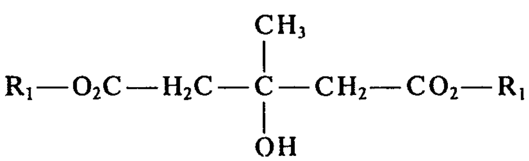
(IV)

produit de formule IV que l'on transforme en acide de formule I par saponification ou décomposition thermique éventuellement catalysée.

2. Procédé selon la revendication 1 de préparation de l'acide 3-méthyl 3-hydroxy glutarique, caractérisé en ce que l'on fait réagir un produit de formule IIa:

$$CH_3-CO-R_a \qquad \text{(IIa)}$$

dans laquelle $R_a$ représente un radical alkoxy ayant de 1 à 4 atomes de carbone, un radical alkanoyloxy ayant de 2 à 4 atomes de carbone ou un atome d'halogène, avec au moins deux équivalents d'un produit de formule IIIa:

$$X'-CH_2-CO_2-R_{1a} \qquad \text{(IIIa)}$$

dans laquelle X' représente un atome de lithium et $R_{1a}$ représente un radical alkyle ayant de 1 à 4 atomes de carbone pour obtenir un produit de formule IVa:

$$\begin{array}{c} CH_3 \\ | \\ R_{1a}-O_2C-CH_2-C-CH_2-CO_2-R_{1a} \\ | \\ OH \end{array} \qquad (IVa)$$

produit de formule IVa que l'on transforme en acide de formule I par saponification ou décomposition thermique avec catalyse acide.

3. Procédé selon la revendication 2 de préparation de l'acide 3-méthyl 3-hydroxy glutarique de formule I, caractérisé en ce que l'on met en oeuvre le procédé en utilisant un produit de formule IIa dans laquelle $R_a$ représente un radical méthoxy, éthoxy, ou acétyloxy ou un atome de chlore et un produit de formule IIIa dans laquelle X' représente un atome de lithium et $R_{1a}$ représente un radical méthyle, éthyle ou tert-butyle et en ce que la saponification du produit de formule IVa obtenu est réalisée en présence de soude aqueuse.

4. Procédé selon l'une quelconque des revendications 2 ou 3, caractérisé en ce que l'on met en oeuvre le procédé en utilisant un produit de formule IIa dans laquelle $R_a$ représente un radical acétyloxy ou un atome de chlore et un produit de formule IIIa dans laquelle X' représente un atome de lithium et $R_{1a}$ représente un radical tert-butyle.

5. Procédé selon l'une quelconque des revendications 2 à 4, caractérisé en ce que la réaction du produit de formule IIa avec le produit de formule IIIa est effectuée à une température comprise entre 0° et −80° C dans un solvant ou un mélange de solvants choisis dans le groupe formé par le tétrahydrofuranne, le diméthyl formamide, l'hexaméthylphosphotriamide et le méthylcyclohexane.

6. Procédé selon l'une quelconque des revendications 2 à 5, caractérisé en ce que le produit de formule III:

$$X-CH_2-CO_2-R_1 \qquad (III)$$

dans laquelle X et $R_1$ ont la signification indiquée à la revendication 1, ou le produit de formule IIIa:

$$X'-CH_2-CO_2-R_{1a} \qquad (IIIa)$$

dans laquelle X' et $R_{1a}$ ont la signification indiquée à la revendication 2, sont préparés par action, dans un solvant, d'une base forte sur l'acétate d'alkyle correspondant et en ce que le produit de formule III ou IIIa obtenu en solution est mis directement en présence du produit de formule II ou IIa.

7. Procédé selon l'une quelconque des revendications 1 à 6, caractérisé en ce que l'on ajoute le produit de formule II ou IIa dans la solution constituée par le produit de formule III ou IIIa dans un solvant ou un mélange de solvants choisis dans le groupe formé par le tétrahydrofurane, le diméthyl formamide, l'hexaméthylphosphotriamide, le méthylcyclohexane.

**Patentansprüche für die Vertragsstaaten: BE, CH, DE, GB, IT, LI, NL, SE**

1. Verfahren zur Herstellung von 3-Methyl-3-hydroxyglutarsäure der Formel I

$$\begin{array}{c} CH_3 \\ | \\ HO_2C-CH_2-C-CH_2-CO_2H \\ | \\ OH \end{array} \qquad (I)$$

dadurch gekennzeichnet, daß man ein Produkt der Formel II

$$CH_3-CO-R \qquad (II)$$

worin R einen Alkoxyrest mit 1 bis 6 Kohlenstoffatomen, einen Formyloxyrest, einen Acyloxyrest mit 2 bis 18 Kohlenstoffatomen oder ein Halogenatom bedeutet, mit zumindest zwei Äquivalenten eines Produkts der Formel III

$$X-CH_2-CO_2-R_1 \qquad (III)$$

worin X ein Alkalimetallatom bedeutet und $R_1$ einen Alkylrest mit 1 bis 6 Kohlenstoffatomen darstellt, umsetzt, um ein Produkt der Formel IV

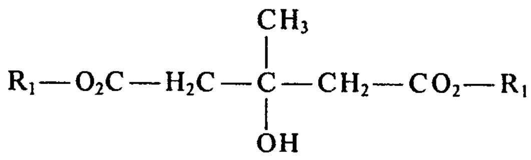

(IV)

zu erhalten, welches Produkt der Formel IV man in die Säure der Formel I durch Verseifen oder durch gegebenenfalls katalysierte thermische Zersetzung überführt.

2. Verfahren gemäß Anspruch 1 zur Herstellung der 3-Methyl-3-hydroxyglutarsäure, dadurch gekennzeichnet, daß man ein Produkt der Formel IIa

$$CH_3-CO-R_a \qquad \text{(IIa)}$$

worin $R_a$ einen Alkoxyrest mit 1 bis 4 Kohlenstoffatomen, einen Alkanoyloxyrest mit 2 bis 4 Kohlenstoffatomen oder ein Halogenatom bedeutet, mit zumindest zwei Äquivalenten eines Produkts der Formel IIIa

$$X'-CH_2-CO_2-R_{1a} \qquad \text{(IIIa)}$$

umsetzt, worin X' ein Lithiumatom bedeutet und $R_{1a}$ einen Alkylrest mit 1 bis 4 Kohlenstoffatomen darstellt, um ein Produkt der Formel IVa

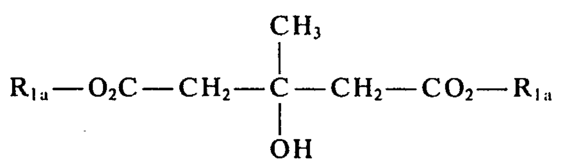

(IVa)

zu erhalten, welches Produkt der Formel IVa man in eine Säure der Formel I durch Verseifung oder thermische Zersetzung unter saurer Katalyse überführt.

3. Verfahren gemäß Anspruch 2, zur Herstellung der 3-Methyl-3-hydroxylglutarsäure der Formel I, dadurch gekennzeichnet, daß man das Verfahren durchführt, indem man ein Produkt der Formel IIa, worin $R_a$ einen Methoxy-, Ethoxy- oder Acetyloxyrest oder ein Chloratom bedeutet, und ein Produkt der Formel IIIa, worin X' ein Lithiumatom bedeutet und $R_{1a}$ einen Methyl-, Ethyl- oder tert.-Butylrest darstellt, einsetzt und die Verseifung des erhaltenen Produktes der Formel IVa in Gegenwart von wäßriger Natronlauge durchführt.

4. Verfahren gemäß einem der Ansprüche 2 oder 3, dadurch gekennzeichnet, daß man das Verfahren durchführt, indem man ein Produkt der Formel IIa, worin $R_a$ einen Acetyloxyrest oder ein Chloratom bedeutet, und ein Produkt der Formel IIIa, worin X' ein Lithiumatom bedeutet und $R_{1a}$ einen tert.-Butylrest darstellt, einsetzt.

5. Verfahren gemäß einem der Ansprüche 2 bis 4, dadurch gekennzeichnet, daß man die Umsetzung des Produkts der Formel IIa mit dem Produkt der Formel IIIa bei einer Temperatur zwischen 0 und $-80°C$ in einem Lösungsmittel oder einem Gemisch von Lösungsmitteln, ausgewählt unter Tetrahydrofuran, Dimethylformamid, Hexamethylphosphortrisamid und Methylcyclohexan, durchführt.

6. Verfahren gemäß einem der Ansprüche 1 bis 5, dadurch gekennzeichnet, daß das Produkt der Formel III

$$X-CH_2-CO_2-R_1 \qquad \text{(III)}$$

worin X und $R_1$ die in Anspruch 1 angegebene Bedeutung besitzen, oder das Produkt der Formel IIIa

$$X'-CH_2-CO_2-R_{1a} \qquad \text{(IIIa)}$$

worin X' und $R_{1a}$ die in Anspruch 2 angegebene Bedeutung besitzen, durch Umsetzung in einem Lösungsmittel, einer starken Base mit dem entsprechenden Alkylacetat hergestellt wird und daß das in Lösung erhaltene Produkt der Formel III oder IIIa direkt mit dem Produkt der Formel II oder IIa zusammengebracht wird.

7. Verfahren gemäß einem der Ansprüche 1 bis 6, dadurch gekennzeichnet, daß man das Produkt der Formel II oder IIa in die aus dem Produkt der Formel III oder IIIa in einem Lösungsmittel oder einem Gemisch von Lösungsmitteln, ausgewählt unter Tetrahydrofuran, Dimethylformamid, Hexamethylphosphortrisamid und Methylcyclohexan, bestehende Lösung zugibt.

8. Als neues, industrielles Produkt der 3-Methyl-3-hydroxyglutarsäure-di-tert.-butylester.

**Patentansprüche für den Vertragsstaat: AT**

1. Verfahren zur Herstellung von 3-Methyl-3-hydroxyglutarsäure der Formel I

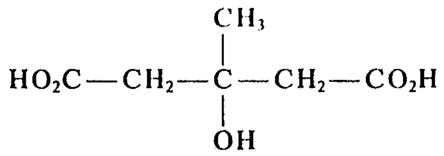

(I)

dadurch gekennzeichnet, daß man ein Produkt der Formel II

$CH_3-CO-R$ (II)

worin R einen Alkoxyrest mit 1 bis 6 Kohlenstoffatomen, einen Formyloxyrest, einen Acyloxyrest mit 2 bis 18 Kohlenstoffatomen oder ein Halogenatom bedeutet, mit zumindest zwei Äquivalenten eines Produkts der Formel III

$X-CH_2-CO_2-R_1$ (III)

worin X ein Alkalimetallatom bedeutet und $R_1$ einen Alkylrest mit 1 bis 6 Kohlenstoffatomen darstellt, umsetzt, um ein Produkt der Formel IV

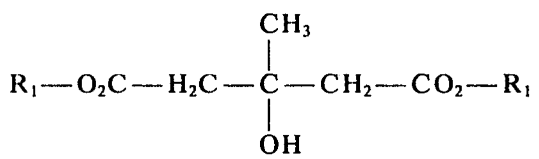

(IV)

zu erhalten, welches Produkt der Formel IV man durch Verseifung oder gegebenenfalls katalysierte thermische Zersetzung in eine Säure der Formel I überführt.

2. Verfahren gemäß Anspruch 1 zur Herstellung der 3-Methyl-3-hydroxyglutarsäure, dadurch gekennzeichnet, daß man ein Produkt der Formel IIa

$CH_3-CO-R_a$ (IIa)

worin $R_a$ einen Alkoxyrest mit 1 bis 4 Kohlenstoffatomen, einen Alkanoyloxyrest mit 2 bis 4 Kohlenstoffatomen oder ein Halogenatom bedeutet, mit zumindest zwei Äquivalenten eines Produkts der Formel IIIa

$X'-CH_2-CO_2-R_{1a}$ (IIIa)

worin X' ein Lithiumatom bedeutet und $R_{1a}$ einen Alkylrest mit 1 bis 4 Kohlenstoffatomen darstellt, umsetzt, um ein Produkt der Formel IVa

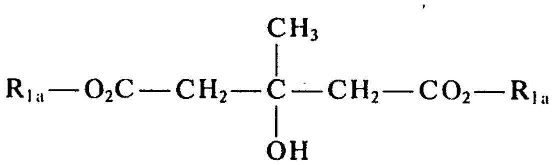

(IVa)

zu erhalten, welches Produkt der Formel IVa man durch Verseifung oder thermische Zersetzung unter saurer Katalyse in eine Säure der Formel I überführt.

3. Verfahren gemäß Anspruch 2, zur Herstellung der 3-Methyl-3-hydroxylglutarsäure der Formel I, dadurch gekennzeichnet, daß man das Verfahren durchführt, indem man ein Produkt der Formel IIa, worin $R_a$ einen Methoxy-, Ethoxy- oder Acetyloxyrest oder ein Chloratom bedeutet, und ein Produkt der Formel IIIa, worin X' ein Lithiumatom bedeutet und $R_{1a}$ einen Methyl-, Ethyl- oder tert.-Butylrest darstellt, einsetzt und daß man die Verseifung des erhaltenen Produktes der Formel IVa in Gegenwart von wäßriger Natronlauge durchführt.

4. Verfahren gemäß einem der Ansprüche 2 oder 3, dadurch gekennzeichnet, daß man das Verfahren durchführt, indem man ein Produkt der Formel IIa, worin $R_a$ einen Acetyloxyrest oder ein Chloratom bedeutet, und ein Produkt der Formel IIIa, worin X' ein Lithiumatom bedeutet und $R_{1a}$ einen tert.-Butylrest darstellt, einsetzt.

5. Verfahren gemäß einem der Ansprüche 2 bis 4, dadurch gekennzeichnet, daß man die Umsetzung des Produkts der Formel IIa mit dem Produkt der Formel IIIa bei einer Temperatur zwischen 0 und

−80°C in einem Lösungsmittel oder einem Gemisch von Lösungsmitteln, ausgewählt unter Tetrahydrofuran, Dimethylformamid, Hexamethylphosphortrisamid und Methylcyclohexan, durchführt.

6. Verfahren gemäß einem der Ansprüche 1 bis 5, dadurch gekennzeichnet, daß das Produkt der Formel III

$$X-CH_2-CO_2-R_1 \qquad (III)$$

worin X und $R_1$ die in Anspruch 1 angegebene Bedeutung besitzen, oder das Produkt der Formel IIIa

$$X'-CH_2-CO_2-R_{1a} \qquad (IIIa)$$

worin X' und $R_{1a}$ die in Anspruch 2 angegebene Bedeutung besitzen, durch Umsetzung in einem Lösungsmittel einer starken Base mit dem entsprechenden Alkylacetat hergestellt wird und daß das in Lösung erhaltene Produkt der Formel III oder IIIa direkt mit dem Produkt der Formel II oder IIa zusammengebracht wird.

7. Verfahren gemäß einem der Ansprüche 1 bis 6, dadurch gekennzeichnet, daß man das Produkt der Formel II oder IIa in die aus dem Produkt der Formel III oder IIIa in einem Lösungsmittel oder einem Gemisch von Lösungsmitteln, ausgewählt unter Tetrahydrofuran, Dimethylformamid, Hexamethylphosphortrisamid und Methylcyclohexan, bestehende Lösung zugibt.

**Claims for the contracting states: BE, CH, DE, GB, IT, LI, NL, SE**

1. Preparation process of 3-methyl 3-hydroxy glutaric acid with the formula I:

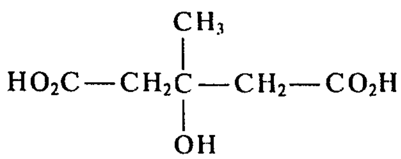

(I)

characterized in that a product with the formula II:

$$CH_3-CO-R \qquad (II)$$

in which R represents an alkoxy radical having from 1 to 6 carbon atoms, a formyloxy radical, an acyloxy radical having from 2 to 18 carbon atoms or a halogen atom, is made to react with at least two equivalents of a product with the formula III:

$$X-CH_2-CO_2-R_1 \qquad (III)$$

in which X represents an alkali metal atom and $R_1$ represents an alkyl radical having from 1 to 6 carbon atoms, so as to obtain a product with the formula IV:

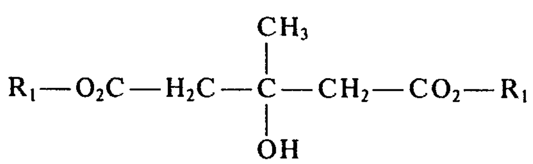

(IV)

which product with the formula IV is transformed into an acid with the formula I by saponification or thermal decomposition, possibly catalysed.

2. Process according to claim 1 for the preparation of 3-methyl-3 hydroxy glutaric acid, characterized in that a product with the formula IIa:

$$CH_3-CO-R_a \qquad (IIa)$$

in which $R_a$ represents an alkoxy radical having from 1 to 4 carbon atoms, an alkanoyloxy radical having from 2 to 4 carbon atoms or a halogen atom, is made to react with at least two equivalents of a product with the formula IIIa:

$$X'-CH_2-CO_2-R_{1a} \qquad (IIIa)$$

in which X′ represents a lithium atom and $R_{1a}$ represents an alkyl radical having from 1 to 4 carbon atoms to obtain a product with the formula IVa:

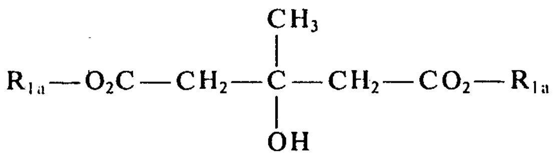 (IVa)

which product with the formula IVa is converted into an acid with the formula I by saponification or thermal decomposition with acid catalysis.

3. Process according to claim 2 for the preparation of 3-methyl-3-hydroxy glutaric acid with the formula I, characterized in that the process is carried out using a product with the formula IIa in which Ra represents a methoxy, ethoxy or acetyloxy radical, or a chlorine atom and a product with the formula IIIa in which X′ represents a lithium atom and $R_{1a}$ represents a methyl, ethyl or tert-butyl radical and in that the saponification of the product with the formula IVa obtained is carried out in the presence of aqueous sodium hydroxide.

4. Process according to any one of the claims 2 or 3, characterized in that the process is carried out by using a product with the formula IIa in which $R_a$ represents an acetyloxy radical or a chlorine atom and a product with the formula IIIa in which X′ represents a lithium atom and $R_{1a}$ represents a tert-butyl radical.

5. Process according to any one of the claims 2 to 4, characterized in that the reaction of the product with the formula IIa with the product with the formula IIIa is carried out at a temperature between 0° and −80°C in a solvent or a mixture of solvents chosen from the group formed by tetrahydrofuran, dimethyl formamide, hexamethylphosphotriamide and methylcyclohexane.

6. Process according to any one of the claims 1 to 5, characterized in that the product with the formula III:

$$X-CH_2-CO_2-R_1 \qquad (III)$$

in which X and $R_1$ have the significance indicated in claim 1, or the product with the formula IIIa:

$$X'-CH_2-CO_2-R_{1a} \qquad (IIIa)$$

in which X′ and $R_{1a}$ have the significance indicated in claim 2, is prepared by the action, in a solvent, of a strong base on the corresponding alkyl acetate and in that the product with the formula III or IIIa obtained in solution is put directly in the presence of the product with the formula II or IIa.

7. Process according to any one of the claims 1 to 6, characterized in that the product with the formula II or IIa is added to the solution composed of the product with the formula III or IIIa in a solvent or a mixture of solvents chosen from the group formed by tetrahydrofuran, dimethyl formamide, hexamethylphosphotriamide, methylcyclohexane.

8. As a new industrial product, the di-tert-butyl ester of 3-methyl-3-hydroxy glutaric acid.

**Claims for the contracting state: AT**

1. Preparation process of 3-methyl 3-hydroxy glutaric acid with the formula I:

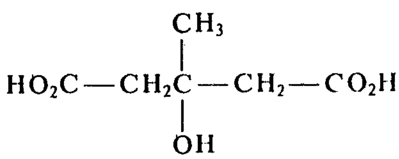 (I)

characterized in that a product with the formula II:

$$CH_3-CO-R \qquad (II)$$

in which R represents an alkoxy radical having from 1 to 6 carbon atoms, a formyloxy radical, an acyloxy radical having from 2 to 18 carbon atoms or a halogen atom, is made to react with at least two equivalents of a product with the formula III:

$$X-CH_2-CO_2-R_1 \qquad (III)$$

in which X represents an alkali metal atom and $R_1$ represents an alkyl radical having from 1 to 6 carbon atoms, so as to obtain a product with the formula IV:

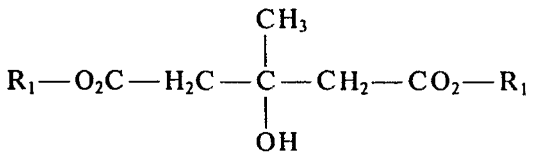

(IV)

which product with the formula IV is transformed into an acid with the formula I by saponification or thermal decomposition, possibly catalysed.

2. Process according to claim 1 for the preparation of 3-methyl 3-hydroxy glutaric acid, characterized in that a product with the formula IIa:

$$CH_3-CO-R_a \quad \text{(IIa)}$$

in which $R_a$ represents an alkoxy radical having from 1 to 4 carbon atoms, an alkanoyloxy radical having from 2 to 4 carbon atoms or a halogen atom, is made to react with at least two equivalents of a product with the formula IIIa:

$$X'-CH_2-CO_2-R_{1a} \quad \text{(IIIa)}$$

in which X' represents a lithium atom and $R_{1a}$ represents an alkyl radical having from 1 to 4 carbon atoms to obtain a product with the formula IVa:

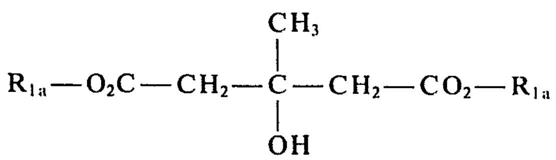

(IVa)

which product with the formula IVa is converted into an acid with the formula I by saponification or thermal decomposition with acid catalysis.

3. Process according to claim 2 for the preparation of 3-methyl-3-hydroxy glutaric acid with the formula I, characterized in that the process is carried out using a product with the formula IIa in which Ra represents a methoxy, ethoxy or acetyloxy radical, or a chlorine atom and a product with the formula IIIa in which X' represents a lithium atom and $R_{1a}$ represents a methyl, ethyl or tert-butyl radical and in that the saponification of the product with the formula IVa obtained is carried out in the presence of aqueous sodium hydroxide.

4. Process according to any one of the claims 2 or 3, characterized in that the process is carried out by using a product with the formula IIa in which $R_a$ represents an acetyloxy radical or a chlorine atom and a product with the formula IIIa in which X' represents a lithium atom and $R_{1a}$ represents a tert-butyl radical.

5. Process according to any one of the claims 2 to 4, characterized in that the reaction of the product with the formula IIa with the product with the formula IIIa is carried out at a temperature between 0° and −80°C in a solvent or a mixture of solvents chosen from the group formed by tetrahydrofuran, dimethyl formamide, hexamethylphosphotriamide and methylcyclohexane.

6. Process according to any one of the claims 1 to 5, characterized in that the product with the formula III:

$$X-CH_2-CO_2-R_1 \quad \text{(III)}$$

in which X and $R_1$ have the significance indicated in claim 1, or the product with the formula IIIa:

$$X'-CH_2-CO_2-R_{1a} \quad \text{(IIIa)}$$

in which X' and $R_{1a}$ have the significance indicated in claim 2, is prepared by the action, in a solvent, of a strong base on the corresponding alkyl acetate and in that the product with the formula III or IIIa obtained in solution is put directly in the presence of the product with the formula II or IIa.

7. Process according to any one of the claims 1 to 6, characterized in that the product with the formula II or IIa is added to the solution composed of the product with the formula III or IIIa in a solvent or a mixture of solvents chosen from the group formed by tetrahydrofuran, dimethyl formamide, hexamethylphosphotriamide, methylcyclohexane.